(19) Europäisches Patentamt

European Patent Office

Office européen des brevets

(11) **EP 3 981 378 A1**

(12) **EUROPÄISCHE PATENTANMELDUNG**

(43) Veröffentlichungstag:
**13.04.2022 Patentblatt 2022/15**

(21) Anmeldenummer: 20200973.4

(22) Anmeldetag: **09.10.2020**

(51) Internationale Patentklassifikation (IPC):
*A61K 8/06* (2006.01)     *A61K 8/37* (2006.01)
*A61K 8/68* (2006.01)     *A61K 8/63* (2006.01)
*A61Q 19/00* (2006.01)     *A61Q 19/08* (2006.01)

(52) Gemeinsame Patentklassifikation (CPC):
**A61K 8/062; A61K 8/375; A61K 8/63; A61K 8/68;
A61Q 19/007; A61Q 19/08**

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**
Benannte Validierungsstaaten:
**KH MA MD TN**

(71) Anmelder: **Evonik Operations GmbH
45128 Essen (DE)**

(72) Erfinder:
• **MACZKIEWITZ, Ursula
45219 Essen (DE)**
• **MEYER, Juergen
45259 Essen (DE)**

• **MAUS, Lisa
47228 Duisburg (DE)**
• **POLAK, Gabriele
58099 Hagen (DE)**
• **BLASKO-BEGOIHN, Heike
45359 Essen (DE)**
• **VON HOF, Jan Marian
44789 Bochum (DE)**

(74) Vertreter: **Evonik Patent Association
c/o Evonik Industries AG
IP Management
Bau 1042A/PB 15
Paul-Baumann-Straße 1
45772 Marl (DE)**

(54) **ZUSAMMENSETZUNGEN ENTHALTEND CERAMID, POLYGLYCERINCARBONSÄUREESTER UND CHOLESTEROL**

(57) Gegenstand der Erfindung sind Zusammensetzungen enthaltend mindestens ein Ceramid, mindestens einen Polyglycerincarbonsäureester und Cholesterol in bestimmten Gewichtsverhältnissen.

**EP 3 981 378 A1**

**Beschreibung**

Gebiet der Erfindung

Gegenstand der Erfindung sind Zusammensetzungen enthaltend mindestens ein Ceramid, mindestens einen Polyglycerincarbonsäureester und Cholesterol in bestimmten Gewichtsverhältnissen.

Stand der Technik

EP3117821 offenbart ein Verfahren zur Herstellung einer Ceramid-Dispersionszusammensetzung, bei dem eine Mischung aus Ceramid, nichtionischem Tensid und mehrwertigem Alkohol auf eine Temperatur von gleich oder höher als 100 ° C erhitzt wird.

EP2295032 offenbart Ceramid-Dispersionen, umfassend natürliches Ceramid und mindestens ein Tensid, wobei die Ceramid-Dispersionen in dem mindestens einen Tensid mindestens einen Polyglycerinfettsäureester mit einem HLB von 10 bis 16 enthalten.

EP975325 offenbart eine Zusammensetzung zur topischen Anwendung, enthaltend eine Kombination einer freien Sphingoidbase und eines Ceramids.

US8710034 offenbart Zusammensetzungen zur Verbesserung der Barrierefunktion der Haut enthaltend Ceramid und Cholesterol.

US2006198800A1 offenbart eine Hautpflegezusammensetzung, umfassend: eine sichere und wirksame Menge von mindestens einem Antifaltenmittel und eine sichere und wirksame Menge eines natürlichen Peeling-Komplexes.

Aufgabe der Erfindung war es, ceramidhaltige Zusammensetzungen bereitzustellen, welche die kosmetischen Eigenschaften-der enthaltenen Ceramide weiter verbessert.

Beschreibung der Erfindung

Überraschenderweise wurde gefunden, dass die in Anspruch 1 beschriebene Zusammensetzung die der Erfindung gestellte Aufgabe zu lösen vermag.

Gegenstand der vorliegenden Erfindung ist daher eine Zusammensetzung enthaltend mindestens ein Ceramid, mindestens einen Polyglycerincarbonsäureester und Cholesterol in bestimmten Gewichtsverhältnissen.

Ein weiterer Gegenstand der Erfindung ist die Verwendung der erfindungsgemäßen Zusammensetzung zur Erhöhung der Barrierefunktion der Haut.

Ein Vorteil der vorliegenden Erfindung ist es, dass die erfindungsgemäßen Zusammensetzungen eine erhöhte Lagerstabiliät aufweisen, daher sich langsamer in ihrer Beschaffenheit, insbesondere hinsichtlich ihrer Viskosität, verglichen zu ceramidhaltigen Zusammensetzungen nach dem Stand der Technik über die Zeit verändern.

Noch ein Vorteil der vorliegenden Erfindung ist es, dass die erfindungsgemäßen Zusammensetzungen eine höhere Anzahl von Gefrier-Tauschritten ohne signifikante Viskositätseinbuße vertragen, verglichen zu ceramidhaltigen Zusammensetzungen nach dem Stand der Technik.

Ein Vorteil der erfindungsgemäßen Zusammensetzung ist es, dass die Zusammensetzung in Formulierungen überlegene sensorische Eigenschaften aufweist, die zu einem verbesserten Hautgefühl und/oder Haargefühl führen.

Ein weiterer Vorteil der erfindungsgemäßen Zusammensetzung ist es, dass die Zusammensetzung in Formulierungen in vitro humane follikuläre dermale Papillen-Zellen (HFDPCs) zur Proliferation anregen, was in vivo einer Stimulation des Haarwachstums gleichkommt.

Ein weiterer Vorteil der erfindungsgemäßen Zusammensetzung ist es, dass die Zusammensetzung in Formulierungen verglichen zu den Einzelkomponenten eine verbesserte Verteilbarkeit aufweist.

Ein weiterer Vorteil der erfindungsgemäßen Zusammensetzung ist es, dass die Zusammensetzung in Formulierungen verglichen zu den Einzelkomponenten eine verbesserte Absorption aufweist. Ein weiterer Vorteil der erfindungsgemäßen Zusammensetzung ist es, dass die Zusammensetzung in Formulierungen verglichen zu den Einzelkomponenten eine verminderte Öligkeit aufweist.

Ein weiterer Vorteil der erfindungsgemäßen Zusammensetzung ist es, dass die Zusammensetzung in Formulierungen verglichen zu den Einzelkomponenten eine verminderte Wachsigkeit aufweist. Ein weiterer Vorteil der erfindungsgemäßen Zusammensetzung ist es, dass die Zusammensetzung in Formulierungen verglichen zu den Einzelkomponenten eine verbesserte Gleitfähigkeit aufweist.

Ein weiterer Vorteil der erfindungsgemäßen Zusammensetzung ist es, dass die Zusammensetzung in Formulierungen verglichen zu den Einzelkomponenten eine verminderte Klebrigkeit aufweist.

Ein weiterer Vorteil der erfindungsgemäßen Zusammensetzung ist es, dass die Zusammensetzung in Formulierungen verglichen zu den Einzelkomponenten eine verbesserte Seidigkeit/Samtigkeit aufweist.

Ein weiterer Vorteil der erfindungsgemäßen Zusammensetzung ist es, dass sie die Hautrauigkeit und die Hautglätte stärker verbessert als ceramidhaltige Zusammensetzungen nach dem Stand der Technik.

Ein weiterer Vorteil der erfindungsgemäßen Zusammensetzung ist es, dass sie eine durch SDS geschädigte Barriere stärker regenerieren als ceramidhaltige Zusammensetzungen nach dem Stand der Technik.

Unter dem Begriff "Ceramid" im Zusammenhang mit der vorliegenden Erfindung werden acylierte Sphingoidbasen verstanden, wobei die Sphingoidbasen bevorzugt ausgewählt sind aus Sphingosin, Sphinganin, 6-Hydroxysphingosin und Phytosphingosin.

Der "pH-Wert" im Zusammenhang mit der vorliegenden Erfindung ist definiert als der Wert, welcher für die entsprechende Zusammensetzung bei 22 °C nach fünf Minuten Rühren mit einer gemäß ISO 4319 (1977) kalibrierten pH-Elektrode gemessen wird.

Die erfindungsgemäß enthaltenen Polyglycerincarbonsäureester stellen Mischungen von unterschiedlichen Substanzen dar; daher ist dem Fachmann klar, dass die angegebenen Zahlenwerte Mittelwerte über die Mischung darstellen.

Unter dem Begriff "Polyglycerin" im Sinne der vorliegenden Erfindung ist ein Polyglycerin zu verstehen, welches auch Glycerin enthalten kann. Somit ist zur Berechnung von Mengen, Massen und dergleichen gegebenenfalls ein Glycerinanteil mit zu berücksichtigen. Das Polyglycerin stellt aufgrund seiner polymeren Eigenschaft eine statistische Mischung verschiedener Verbindungen dar. Polyglycerin kann ausgebildete Etherbindungen zwischen zwei primären, einer primären und einer sekundären sowie zwei sekundären Positionen der Glycerinmonomere aufweisen. Aus diesem Grund besteht das Polyglycerin-Grundgerüst üblicherweise nicht ausschließlich aus linear verknüpften Glycerin-Einheiten, sondern kann auch Verzweigungen und Cyclen enthalten. Für Details siehe z.B. *"Original synthesis of linear, branched and cyclic oligoglycerol Standards"*, Cassel et al., J. Org. Chem. 2001, 875-896.

Analoges gilt für den Begriff "Polyglycerincarbonsäureester" im Zusammenhang mit der vorliegenden Erfindung. Alle angegebenen Prozent (%) sind, wenn nicht anders angegeben, Massenprozent.

Gegenstand der vorliegenden Erfindung ist somit eine Zusammensetzung enthaltend

A) mindestens ein Ceramid,

B) mindestens einen Polyglycerincarbonsäureester und

C) Cholesterol und/oder mindestens ein Cholesterolderivat ausgewählt aus der Gruppe Cholesterolsulfat, insbesondere das Kaliumsalz des Cholesterolsulfates, das Potassium Cholesteryl Sulfate, Cholesterol Hydrogen Succinate und 7-Dehydrocholesterol, bevorzugt in einer Gesamtmenge von 0,1 Gew.-% bis 3,0 Gew.-%, bevorzugt von 0,1 Gew.-% bis 2,0 Gew.-%, besonders bevorzugt von 0,2 Gew.-% bis 1,0 Gew.-%, wobei sich die Gewichtsprozente auf die Gesamtzusammensetzung beziehen,

dadurch gekennzeichnet, dass

das Gewichtsverhältnis aller enthaltenen Ceramide zur Summe des enthaltenen Cholesterols und/oder des mindestens einen Cholesterolderivates von 1:0,1 bis 1:0,9, bevorzugt 1:0,1 bis 1:0,8, besonders bevorzugt von 1:0,2 bis 1:0,7, beträgt

Eine bevorzugte erfindungsgemäße Zusammensetzung ist dadurch gekennzeichnet, dass das Gewichtsverhältnis aller enthaltenen Ceramide zu allen enthaltenen Polyglycerincarbonsäureestern von 1:1 bis 1:30, bevorzugt von 1:1 bis 1:25, besonders bevorzugt von-1:2 bis 1:20, beträgt.

Es ist erfindungsgemäß bevorzugt, dass die erfindungsgemäße Zusammensetzung dadurch gekennzeichnet ist, dass sie

D) mindestens eine Sphingoidbase, bevorzugt ausgewählt aus der Gruppe Sphingosin, Sphinganin, 6-Hydroxysphingosin, N-Acetylphytosphingosine und Phytosphingosin, insbesondere Phytosphingosin, enthält.

Komponente D) ist erfindungsgemäß bevorzugt bezogen auf die gesamte erfindungsgemäße Zusammensetzung in einer Menge von 0,01 Gew.-% bis 2,0 Gew.-%, bevorzugt 0,02 Gew.-% bis 1 Gew.-%, besonders bevorzugt 0,02 Gew.-% bis 0,8 Gew.-%, enthalten.

Erfindungsgemäß bevorzugt beträgt das Gewichtsverhältnis der Komponente A) zu Komponente D) in der erfindungsgemäßen Zusammensetzung in der von 1:1 bis 1: 0,01, bevorzugt von 1: 0,8 bis 1: 0,02, besonders bevorzugt von 1: 0,7 bis 1: 0,05.

Es ist erfindungsgemäß bevorzugt, dass die erfindungsgemäße Zusammensetzung dadurch gekennzeichnet ist, dass sie

E) mindestens eine freie Fettsäure, bevorzugt ausgewählt aus der Gruppe der Fettsäuren mit einer Kettenlänge von 12 bis 40, bevorzugt 14 bis 24, besonders bevorzugt 16 bis 22, C-Atomen, enthält.

Komponente E) ist erfindungsgemäß bevorzugt bezogen auf die gesamte erfindungsgemäße Zusammensetzung in einer Menge von 0,01 Gew.-% bis 3,0 Gew.-%, bevorzugt 0,05 Gew.-% bis 2,0 Gew.-%, besonders bevorzugt 0,1 Gew.-% bis 1,0 Gew.-%, enthalten.

Erfindungsgemäß bevorzugt beträgt das Gewichtsverhältnis der Komponente B) zu Komponente E) in der erfindungsgemäßen Zusammensetzung in der von 1: 0,001 bis 1:1, bevorzugt von 1: 0,01 bis 1: 0,5, besonders bevorzugt von 1: 0,02 bis 1: 0,1.

Es ist erfindungsgemäß bevorzugt, dass die erfindungsgemäße Zusammensetzung dadurch gekennzeichnet ist, dass sie bezogen auf die Gesamtzusammensetzung 20 Gew.-% bis 99 Gew.-%, bevorzugt 40 Gew.-% bis 97 Gew.-%, besonders bevorzugt 50 Gew.-% bis 95 Gew.-%, Wasser enthält.

Bevorzugte erfindungsgemäße Zusammensetzungen stellen Wirkstoffkonzentrate dar, welche einen hohen Aktivgehalt aufweisen; solch eine bevorzugte erfindungsgemäße Zusammensetzung ist dadurch gekennzeichnet, dass sie bezogen auf die Gesamtzusammensetzung Komponente A), B) und C) in einer Gesamtmenge von 1,0 Gew.-% bis 25 Gew.-%, bevorzugt 2,0, Gew.-% bis 20 Gew.-%, besonders bevorzugt 4,0 Gew.-% bis 15 Gew.-%, enthält

Erfindungsgemäß bevorzugte Zusammensetzungen enthalten mindestens zwei Ceramide, bevorzugt mindestens drei Ceramide, besonders bevorzugt genau drei Ceramide.

Bevorzugt in der erfindungsgemäßen Zusammensetzung enthaltene Ceramide sind ausgewählt aus der Gruppe Ceramid NP, Ceramid AP, Ceramid EOP, Ceramid NDS, Ceramid ADS, Ceramid EODS, Ceramid NS, Ceramid AS, Ceramid EOS, Ceramid NH, Ceramid AH und Ceramid EOH, bevorzugt ausgewählt aus der Gruppe Ceramide NP, Ceramide AP, Ceramid NS, Ceramide EOP und Ceramide EOS.

Erfindungsgemäß bevorzugte Zusammensetzungen sind dadurch gekennzeichnet, dass der Polyglycerincarbonsäureester der Komponente B) aus einem Polyglycerin zusammengesetzt ist, welches einen Polymerisationsgrad von 2,0 bis 25, bevorzugt von 2,5 bis 20, besonders bevorzugt von 3,0 bis 15, aufweist.
Der Polymerisationsgrad des Polyglycerins $N$ wird über dessen Hydroxylzahl (OHV, in mg KOH/g) gemäß folgender Formel berechnet:

$$N = \frac{(112200 - 18 \cdot OHV)}{(74 \cdot OHV - 56100)}$$

Geeignete Bestimmungsmethoden zur Ermittlung der Hydroxylzahl sind insbesondere solche gemäß DGF C-V 17 a (53), Ph. Eur. 2.5.3 Method A und DIN 53240.

Die Zusammensetzung der vorliegenden Erfindung ist bevorzugt dadurch gekennzeichnet, dass der Polyglycerincarbonsäureester der Komponente B) aus mindestens einer Carbonsäure ausgewählt aus Fettsäuren, insbesondere aufweisend 12 bis 26, bevorzugt 14 bis 24, besonders bevorzugt 16 bis 22, Kohlenstoffatome, zusammengesetzt ist. Die Fettsäuren sind in der Regel unverzweigt und bestehen aus einer geraden Anzahl Kohlenstoffatomen. Eventuelle Doppelbindungen besitzen cis-Konfiguration. Beispiele für Fettsäuren sind: Capronsäure, Caprylsäure, Caprinsäure, Laurinsäure, Myristinsäure, Palmitinsäure, Palmitoleinsäure, Isostearinsäure, Stearinsäure, 12 Hydroxystearinsäure, Dihydroxystearinsäure, Ölsäure, Linolsäure, Linolensäure, Petrolesinsäure, Elaidinsäure, Arachinsäure, Behensäure, Erucasäure, Gadoleinsäure, Linolensäure, Eicosapentaensäure, Docosahexaensäure und Arachidonsäure.
Die mindestens eine Carbonsäure, aus welche der Polyglycerincarbonsäureester der Komponente B) erfindungsgemäß zusammengesetzt ist, ist insbesondere bevorzugt ausgewählt aus Palmitinsäure, Stearinsäure und Behensäure.

Es ist insbesondere erfindungsgemäß bevorzugt, dass der Polyglycerincarbonsäureester der Komponente B) aus Mischungen von Fettsäuren zusammengesetzt ist, insbesondere aus Mischungen von mindestens zwei ausgewählt aus Palmitinsäure, Stearinsäure und Behensäure, wobei die Summe dieser mindestens 60 Gew.-%, bevorzugt mindesten 75 Gew.-%, noch mehr bevorzugt mindestens 85 Gew.-% bezogen auf alle in der Mischung enthaltenen Fettsäuren ausmacht. In diesem Zusammenhang ist es insbesondere erfindungsgemäß bevorzugt, wenn das Gewichtsverhältnis von Palmitinsäure zu Stearinsäure zu Behensäure bei 1,0 bis 3,0 zu 1,0 bis 3,0 zu 1,0, bevorzugt von 1,2 bis 2,1 zu 1,4 bis 2,4 zu 1,0, liegt.

Der erfindungsgemäß in der Komponente B) enthaltene Polyglycerincarbonsäureester kann erfindungsgemäß durch Abmischen von verschiedenen, separat erhaltenen Polyglycerincarbonsäurestern bereitgestellt werden. Also beispielsweise durch Abmischen eines Polyglycerin-Palmitats mit einem Polyglycerin-Behenat.

Der erfindungsgemäß in der Komponente B) enthaltene Polyglycerincarbonsäureester ist erfindungsgemäß bevorzugt dadurch gekennzeichnet, dass er eine Verseifungszahl von 20 bis 199 mg KOH / g, bevorzugt von 30 bis 100 mg KOH / g, besonders bevorzugt von 40 bis 70 mg KOH / g, aufweist. Die Bestimmung der Verseifungszahl erfolgt fachmännisch in Anlehnung an DGF C-V 3 oder DIN EN ISO 3681.

Bevorzugte erfindungsgemäße Zusammensetzungen sind insbesondere Formulierungen, insbesondere in Form einer kosmetischen, pharmazeutischen oder dermatologischen Formulierung.
Aus den oben beschriebenen Wirkstoffkonzentraten lassen sich durch Abmischung Formulierungen herstellen, bei denen die Wirkstoffkonzentration im Vergleich zu den vorgenannten Konzentraten erniedrigt ist; solch eine alternative, bevorzugte erfindungsgemäße Zusammensetzung ist dadurch gekennzeichnet, dass sie bezogen auf die Gesamtzusammensetzung Komponente A), B) und C) in einer Gesamtmenge von 0,01 Gew.-% bis 1,25 Gew.-%, bevorzugt 0,01 Gew.-% bis 1,00 Gew.-%, besonders bevorzugt 0,02 Gew.-% bis 1,00 Gew.-%, enthält, und ist insbesondere bevorzugt eine kosmetische, pharmazeutische oder dermatologische Formulierung.

Die erfindungsgemäßen Formulierungen können des Weiteren mindestens eine zusätzliche Komponente enthalten, ausgewählt aus der Gruppe der

Emollients,
Emulgatoren,
Verdicker/Viskositätsregler/Stabilisatoren,
UV-Lichtschutzfilter,
Antioxidantien,
Hydrotrope (oder Polyole),
Fest- und Füllstoffe,
Filmbildner,
Perlglanzadditive,
Deodorant- und Antitranspirantwirkstoffe,
Insektrepellentien,
Selbstbräuner,
Konservierungsstoffe,
Konditioniermittel,
Parfüme,
Farbstoffe,
Geruchsabsorber,
kosmetische Wirkstoffe,
Pflegeadditive,
Überfettungsmittel,
Lösungsmittel.

Substanzen, die als beispielhafte Vertreter der einzelnen Gruppen eingesetzt werden können, sind dem Fachmann bekannt und können beispielsweise der deutschen Anmeldung DE 102008001788.4 entnommen werden. Diese Patentanmeldung wird hiermit als Referenz eingeführt und gilt somit als Teil der Offenbarung.

Bezüglich weiterer fakultativer Komponenten sowie die eingesetzten Mengen dieser Komponenten wird ausdrücklich auf die dem Fachmann bekannten einschlägigen Handbücher, zum Beispiel K. Schrader, "Grundlagen und Rezepturen der Kosmetika", 2. Auflage, Seite 329 bis 341, Hüthig Buch Verlag Heidelberg, verwiesen.

Die Mengen der jeweiligen Zusätze richten sich nach der beabsichtigten Verwendung.

Typische Rahmenrezepturen für die jeweiligen Anwendungen sind bekannter Stand der Technik und sind beispielsweise in den Broschüren der Hersteller der jeweiligen Grund- und Wirkstoffe enthalten. Diese bestehenden Formulierungen können in der Regel unverändert übernommen werden. Im Bedarfsfall können zur Anpassung und Optimierung die gewünschten Modifizierungen aber durch einfache Versuche komplikationslos vorgenommen werden.

Erfindungsgemäß bevorzugte Zusammensetzungen der vorliegenden Erfindung sind Emulsionen, insbesondere Öl-in-Wasser-Emulsionen.

Eine besonders bevorzugte erfindungsgemäße Zusammensetzung ist dadurch gekennzeichnet, dass sie einen pH-Wert im Bereich von 4,0 bis 8,0, bevorzugt von 4,5 bis 7,4 besonders bevorzugt von 5,0 bis 7,2, aufweist.

Ein weiterer Gegenstand der vorliegenden Erfindung ist die Verwendung einer erfindungsgemäßen Zusammensetzung zur Erhöhung der Barrierefunktion der Haut.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

Folgende Abbildungen sind Bestandteil der Beispiele:

Abbildung 1: LDH-Konzentration im Überstand der Hautmodelle, gemessen 24h nach Applikation der Testformulierungen auf die zuvor mit SDS geschädigten Hautmodelle.

Abbildung 2: Interleukin $1\alpha$-Konzentration im Überstand der Hautmodelle, gemessen 24h nach Applikation der Testformulierungen auf die zuvor mit SDS geschädigten Hautmodelle.

Abbildung 3: Das Diagramm zeigt die Differenz des Parameters SESM zum Startwert T0. Eine Abnahme von SESM ist gleichzusetzen mit einer Verbesserung der Hautglätte.

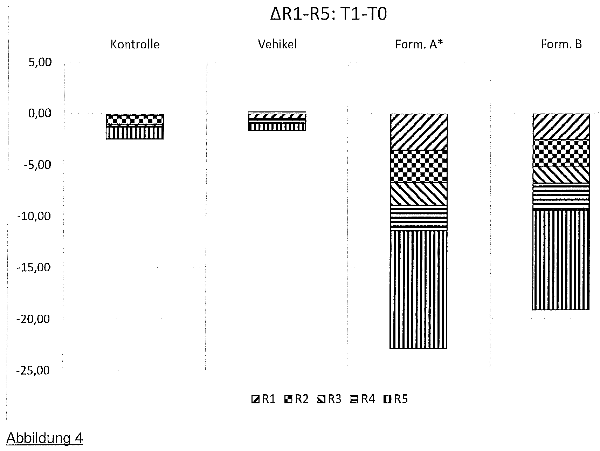

Abbildung 4: Das Diagramm zeigt die Differenz der Rauigheitsparameter zum Startwert T0 nach einwöchiger Applikation der Testformulierungen.

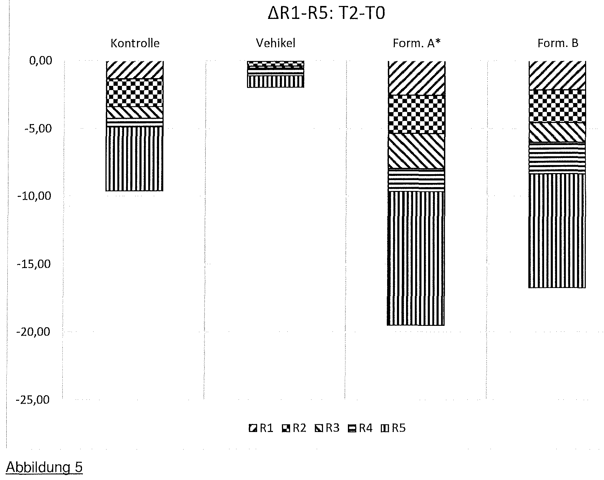

Abbildung 5: Das Diagramm zeigt die Differenz der Rauigheitsparameter zum Startwert T0 nach zweiwöchiger Applikation der Testformulierungen.

Beispiele:

Erfindungsgemäße Beispiele sind mit einem * gekennzeichnet.

*Beispiel 1: Synthese des eingesetzten Polyglycerincarbonsäureesters (Polyglycerin-6 verestert mit C16-, C18- und C22-Carbonsäure)*

Ein Gemisch aus kommerziell erhältlichem Polyglycerin-6 (Firma Spiga Nord S.p.A.; 245,0 g, Hydroxylzahl = 980 mg KOH/g), einem technischen Gemisch aus Stearinsäure und Palmitinsäure (79,0 g, Säurezahl = 207 mg KOH/g, C16 / C18:0 ~ 47:53), Behensäure (28,3 g, Säurezahl = 165,5 mg KOH/g) und $Na_2CO_3$ (2,0 g) wurde innerhalb von 3 h unter Stickstoffeinleitung bis auf 240 °C erhitzt und das Gemisch anschließend so lange bei dieser Temperatur gerührt und das entstehende Wasser kontinuierlich entfernt, bis eine Säurezahl von ≤1,0 erreicht war. Das erhaltende Produkt hatte eine Verseifungszahl von 61 mg KOH/g.

*Beispiel 2: Regenerierung von geschädigter Hautbarriere*

Folgende Formulierung werden eingesetzt, Beispiel A ist erfindungsgemäß, Beispiel B entspricht dem Stand der Technik, wie er beispielsweise in Beispiel 9 der US2006198800 beschrieben ist.

|  | Beispiel A (w/w%) * | Beispiel B (w/w%) |
|---|---|---|
| Polyglycerin aus Beispiel 1 | 6,0 |  |
| Sodium Lauroyl Lactylate |  | 10 |
| Ceramide EOP | 0,001 | 0,001 |
| Ceramide NP | 1 | 1 |
| Ceramide AP | 0,5 | 0,5 |
| Cholesterol | 0,5 | 0,5 |
| Phytosphingosine | 0,5 | 0,5 |
| Behensäure | 0,5 |  |
| Konservierungsmittel | q.s. | q.s |
| Wasser | Ad 100,0 | Ad 100,0 |

Der im folgenden beschriebene Versuch wird auf Epidermis Hautmodellen durchgeführt (EpiCS, SkinInVitro, Troisdorf). Diese dreidimensionalen Hautmodelle bestehen aus einer Epidermis, proliferierenden Keratinozyten und einem Stratum Corneum mit einer intakten Barriere Funktion. Um die regenerierende Wirkung der Testformulierungen auf eine geschädigte Hautbarriere zu untersuchen, werden die Hautmodelle mit 0,325%iger wässriger Natriumdodecylsulfat-Lösung (SDS) für 40 min geschädigt. Anschließend erfolgt die Applikation der Testformulierungen. Als Testformulierungen werden die Beispiel A * und Beispiel B mit einer Konzentration von 0,1, 0,3 und 0,5% in Wasser eingesetzt. Zusätzlich wird ein Vehikel mitgetestet. Hierbei handelt es sich um Ultrapure Water. Die Dauer der Applikation beträgt 1 Stunde.

Nach der Applikation erfolgt ein Medienwechsel. Am nächsten Tag werden die Überstände abgenommen. Diese Überstände werden dazu benutzt, das Enzym Laktatdehydrogenase (LDH) und das Interleukin 1α zu quantifizieren. Das Enzym Laktatdehydrogenase wird u.a. bei einer Zellmembranschädigung freigesetzt. Interleukin 1α ist ein Protein, das bei Entzündungsprozessen von den Zellen exprimiert wird.

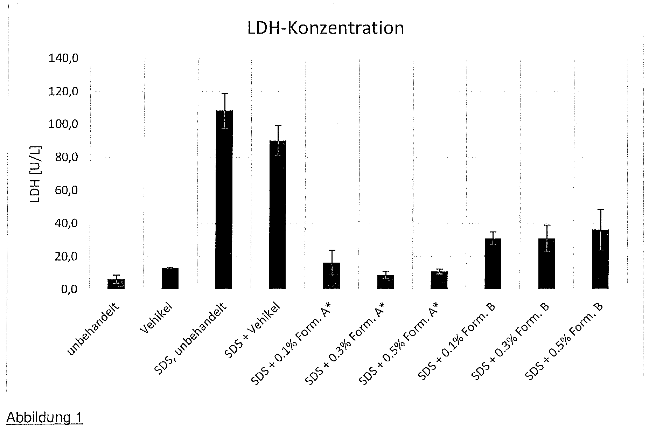

Abbildung 1 zeigt die LDH-Konzentration 24 Stunden nach Applikation der Testformulierungen auf die zuvor mit SDS geschädigten Hautmodelle. Durch die Schädigung der Hautbarriere durch das Natriumdodecylsulfat steigt die Konzentration des Enzyms LDH sehr stark an. Dieser Anstieg wird durch beide Testformulierungen reduziert. Das Vehikel zeigt diesen Effekt nicht (SDS + Vehikel). Man erkennt sehr deutlich, dass die Reduktion durch das erfindungsgemäße Beispiel A stärker ausfällt als durch das Stand der Technik Beispiel B.

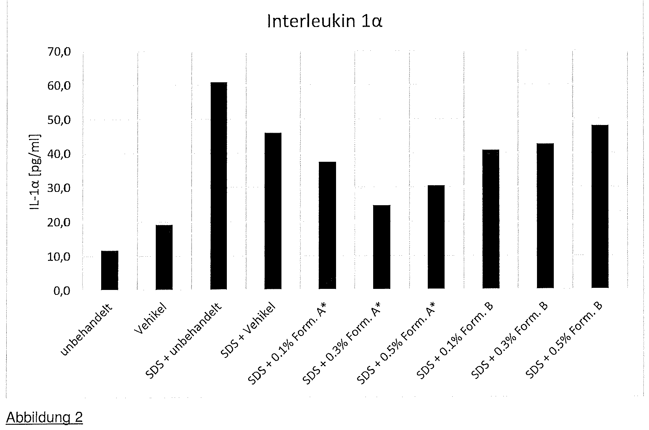

Abbildung 2 zeigt die Konzentration an Interleukin 1α (IL-1α) 24h nach Applikation der Testformulierungen auf die zuvor mit SDS geschädigten Hautmodelle. Durch die Schädigung der Hautbarriere mit SDS wird ein Entzündungsprozess in Gang gesetzt, was sich an dem starken Anstieg der Konzentration von IL-1α zeigt. Das Vehikel (Ultrapure Water) kann diesen Entzündungsprozess nicht stoppen (SDS + Vehikel), während bei den Hautmodellen, die nach der SDS-Schädigung mit den beiden Testformulierungen behandelt worden sind, die Expression des Entzündungsmarkers IL-1$\alpha$ deutlich reduziert ist. Bei Beispiel A geht dies zurück bis auf das Niveau der nicht geschädigten Hautmodelle (Vehikel), bei Testformulierung B wird die Expression von IL-1$\alpha$ ebenfalls deutlich reduziert, sie liegt aber signifikant höher als bei Beispiel A.

Der oben beschriebene Hautmodell-Versuch zeigt, dass beide Testformulierungen in der Lage sind, die Schädigung der Hautbarriere durch Natriumdodecylsulfat zu reparieren, das erfindungsgemäße Beispiel A zeigt hier aber klare Vorteile gegenüber dem Stand der Technik Beispiel B.

*Beispiel 3:* in vivo *Studie zu Hauttrockenheit*

An dieser Studie nehmen 40 Probanden mit trockener Haut teil. Die Probanden erhalten entweder zwei Testformulierungen, die sie auf der linken beziehungsweise rechten Innenseite der Unterarme auftragen müssen, oder sie erhalten eine Testformulierung, die auf einem Unterarm aufgetragen werden muss, während der andere Unterarm unbehandelt bleibt (Kontrolle). Die Testformulierungen beziehungsweise die Kontrolle werden nach dem Zufallsprinzip verteilt, jedoch so, dass alle Testformulierungen beziehungsweise die Kontrolle an 20 Probanden getestet werden. Die Probanden werden angewiesen, während der Studie nur die Testformulierungen zu verwenden. Eine normale Reinigung/Duschen bleibt aber erlaubt.

Die Applikation der Testformulierungen erfolgt über einen Zeitraum von zwei Wochen zweimal täglich. Vor Beginn der Applikation beziehungsweise nach einer und nach zwei Wochen werden Aufnahmen der Unterarminnenseite gemacht. Hierfür wird eine Visioscan VC 98-Kamera der Firma CK electronic GmbH verwendet. Dies ist eine spezielle Kamera, die vergrößerte Schwarz-Weiß-Bilder der Hautoberfläche aufnimmt. Anhand der Graustufenverteilung im Bild werden dann mit Hilfe einer integrierten Software die Rauheitsparameter R1, R2, R3, R4 und R5 und der Parameter SESM (Hautglätte) ermittelt. Dieser Parameter ist proportional zur Breite und Form der Falten. Eine nähere Beschreibung zur Berechnung dieses Parameters findet man im Handbuch der Visioscan VC 98 Kamera, Version "*VC 98 english FireWire 04/2005 DK*".

Der Parameter SESM muss abnehmen, wenn die Hautglätte sich verbessern soll.

Die Rauheitsparameter R1, R2, R3, R4 und R5 kommen ursprünglich aus der Metallindustrie und sind in den Vorschriften DIN 4762-4768 als Ra-Rz festgelegt. In der Visioscan Software werden diese Ergebnisse als Index (in Graustufen) ausgedrückt und lehnen sich an die ursprünglichen Ra-Rz-Werte an. Die Parameter haben folgende Bedeutung:

| Parameter | Bezeichnung | Bezeichnung lt. DIN |
|---|---|---|
| R1 | Rauhtiefe | Rt |
| R2 | Maximale Rauhtiefe | Rmax |
| R3 | Gemittelte Rauhtiefe | Rz |
| R4 | Glättungstiefe | Rp |
| R5 | Arithmetischer Mittenrauhwert | Ra |

Um die Effektivität der Testformulierungen zu ermitteln, wird für jeden Probanden die Differenz der Parameter nach einer beziehungsweise zwei Wochen zum Startwert ermittelt (T1-T0, T2-T0) und der Mittelwert der 20 Probanden berechnet.

Die folgende Tabelle zeigt die Zusammensetzung der Testformulierung. Hierbei handelt es sich um eine O/W-Lotion, die auf handelsüblichen kosmetischen Rohstoffen basiert. Für die Herstellung der Formulierung sind folgende Schritte notwendig:

1. Phase A und B werden auf 70 °C erhitzt.
2. Phase B wird zu Phase A gegeben ohne zu rühren.
3. Anschließend wird homogenisiert.
4. Die Emulsion wird unter Rühren bis auf 60 °C abgekühlt und Phase C wird zugegeben.
5. Es wird ein weiteres Mal für kurze Zeit homogenisiert.
6. Die Emulsion wird unter Rühren weiter abgekühlt bis auf 30 °C. Unterhalb von 40 °C werden Phase D, E und F zugegeben.

Tab. 1: Testformulierungen in-vivo-Studie

| Phase | Inhaltsstoffe | Vehikel | Form. A* | Form. B |
|---|---|---|---|---|
| A | AXOL® C 62 Pellets (Glyceryl Stearate Citrate) | 2,00 | 2,00 | 2,00 |
| | TEGO® Alkanol 1618 (Cetearyl Alcohol) | 1,00 | 1,00 | 1,00 |
| | TEGOSOFT® CT (Caprylic/Capric Triglyceride) | 4,50 | 4,50 | 4,50 |
| | TEGOSOFT® TN (C12-15 Alkyl Benzoate) | 4,50 | 4,50 | 4,50 |
| B | Beispiel A | | 5,00 | |
| | Beispiel B | | | 5,00 |
| | Wasser | 85,20 | 80,20 | 80,20 |
| C | TEGO® Carbomer 141 (Carbomer) | 0,20 | 0,20 | 0,20 |
| | TEGOSOFT® CT (Caprylic/Capric Triglyceride) | 0,80 | 0,80 | 0,80 |
| D | Sodium Hydroxide (10 %) | 0,70 | 0,70 | 0,70 |
| E | Verstatil® PC (Phenoxyethanol; Caprylyl Glycol) | 1,00 | 1,00 | 1,00 |
| F | Perfume | 0,10 | 0,10 | 0,10 |
| | pH | 6,0-6,5 | 6,0-6,5 | 6,0-6,5 |

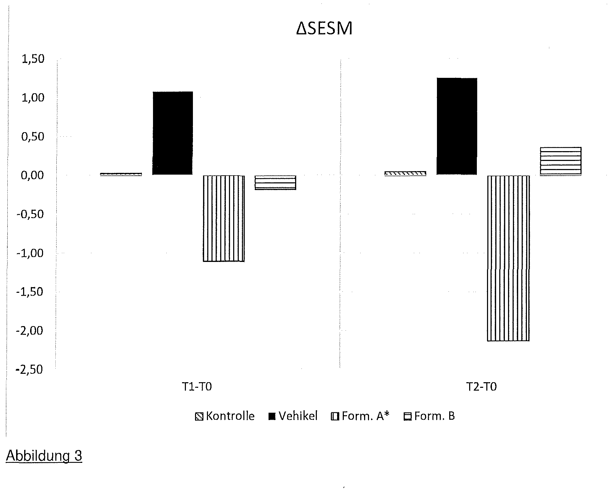

In Abbildung 3 findet man das Ergebnis für die Hautglätte. Das Vehikel, also die Basisformulierung ohne Wirkstoff, ist nicht in der Lage, die Hautglätte zu verbessern, im Gegenteil findet eher eine Verschlechterung statt. Lediglich die Testformulierung, die das erfindungsgemäße Beispiel A enthält, verbessert die Hautglätte, während die Testformulierung mit Beispiel B so gut wie keinen Einfluss auf die Hautglätte hat.

Bei den Rauigkeitsparametern sind beide Testformulierungen in der Lage, die Hautrauigkeit zu verringern, während das Vehikel keine Wirksamkeit zeigt. Sowohl nach einer Woche als auch nach zwei Wochen Applikationsdauer verbessert die erfindungsgemäße Formulierung A die Hautrauigkeit stärker als die Stand der Technik Formulierung B. Zusammenfassung der Studienergebnisse: Zusammengefasst lässt sich an Hand der Ergebnisse der in-vivo-Studie zeigen, dass erfindungsgemäße Beispiel A eine verbesserte Wirksamkeit zeigt im Vergleich zu dem Stand d er Technik Beispiel B bezüglich Verbesserung der Hautglätte und Reduzierung der Hautrauigkeit bei Probanden mit trockener Haut.

Weitere erfindungsgemäße Formulierungen sehen wir folgt aus:

| Phase | Beispiel | Nr 4 | Nr. 5 | Nr. 6 | Nr.7 | Nr.8 | Nr. 9 | Nr. 10 |
|---|---|---|---|---|---|---|---|---|
| | | w/w% | w/w% | w/w% | w/w% | w/w% | w/w% | w/w% |
| A | Polyglycerin aus Beispiel 1 | 6.00 | 6.00 | 6.00 | 6.00 | 6.00 | 6.00 | 6.00 |
| A | Ceramide EOP | 0.10 | 1.45 | 0.10 | 0.16 | 0.10 | 0.99 | 0.11 |
| A | Ceramide EOS | | | | | | 0.33 | |
| A | Ceramide NP | 0.51 | | 0.51 | 0.52 | 0.54 | | 0.66 |
| A | Ceramide AP | 0.41 | | 0.41 | 0.10 | 0.45 | | |
| A | Ceramide NS | 0.18 | | 0.18 | 0.29 | 0.22 | | 0.21 |
| A | Cholesterol | 0.47 | 0.42 | 0.47 | 0.47 | 0.45 | 0.32 | 0.49 |
| A | Potassium Cholesterol Sulfate | 0.11 | | 0.11 | 0.11 | | 0.08 | |
| A | N-Acetylphytosphingosine | | | | 0.10 | | | |
| A | Phytosphingosine | | | | | | | 0.22 |
| A | Pentadecansäure | | | 0.03 | | | | |
| A | Heptadecansäure | | | 0.03 | | | | |
| A | Palmitinsäure | 0.11 | 0.08 | 0.09 | 0.11 | 0.14 | 0.08 | 0.15 |
| A | Stearinsäure | 0.08 | 0.06 | 0.07 | 0.08 | 0.10 | 0.06 | 0.11 |
| A | Ölsäure | 0.08 | 0.06 | 0.07 | 0.08 | 0.10 | 0.06 | 0.11 |
| A | Linolsäure | 0.04 | 0.03 | 0.03 | 0.04 | | 0.03 | |
| A | Behensäure | | | | | 0.12 | 0.08 | |
| A | Carnaubawachssäure | 0.12 | 0.09 | 0.10 | 0.12 | | | 0.14 |
| B | Konservierungsmittel | q.s. | q.s. | q.s. | q.s. | q.s. | q.s. | q.s. |
| C | Wasser | Ad 100.0 | Ad 100.0 | Ad 100.0 | Ad 100.0 | Ad 100.0 | Ad 100.0 | Ad 100.0 |

| Phase | Beispiel | Nr. 11 | Nr. 12 | Nr. 13 | Nr. 14 |
|---|---|---|---|---|---|
| | | w/w% | w/w% | w/w% | w/w% |
| A | Polyglycerin aus Beispiel 1 | 6,0 | 6,0 | 6,0 | 6,0 |
| A | Ceramide EOP | 0,001 | 0,001 | 0,001 | 0,001 |
| A | Ceramide NP | 1 | 1 | 1 | 1 |
| A | Ceramide AP | 0,5 | 0,5 | 0,5 | 0,5 |
| A | Cholesterol | 0,5 | 0,5 | 0,5 | 0,5 |
| A | Phytosphingosine | 0,5 | 0,5 | 0,5 | 0,5 |
| A | Behensäure | 0,5 | 0,5 | 0,5 | 0,5 |
| A | Salicyloyl Phytosphingosine | | | 0,5 | |
| B | Konservierungsmittel | q.s. | q.s. | q.s. | |
| C | Creatine | 1,0 | | | |
| C | Panthenol | | 5,0 | | |
| C | Nicotinamid | | | | 5,0 |
| C | Wasser | Ad 100,0 | Ad 100,0 | Ad 100,0 | Ad 100,0 |
| | | | | | |

| Phase | Beispiel | Nr 15 | Nr 16 | Nr 17 |
|---|---|---|---|---|
| | | w/w% | w/w% | w/w% |
| A | Polyglycerin aus Beispiel 1 | 6.00 | 6.00 | 6.00 |
| A | Ceramide EOP | 0.10 | 0.10 | 0.10 |
| A | Ceramide EOS | | | |
| A | Ceramide NP | 0.51 | 0.51 | 0.51 |
| A | Ceramide AP | 0.41 | 0.41 | 0.41 |
| A | Ceramide NS | 0.18 | 0.18 | 0.18 |
| A | Cholesterol | 0.47 | 0.47 | 0.47 |
| A | Potassium Cholesterol Sulfate | 0.11 | 0.11 | 0.11 |
| A | Palmitinsäure | 0.11 | 0.11 | 0.11 |
| A | Stearinsäure | 0.08 | 0.08 | 0.08 |
| A | Ölsäure | 0.08 | 0.08 | 0.08 |
| A | Linolsäure | 0.04 | 0.04 | 0.04 |
| A | Behensäure | | | |
| A | Carnaubawachssäure | 0.12 | 0.12 | 0.12 |
| A | Tocopherol | 1,0 | | |
| A | Tocopherolacetat | | 1,0 | |

| A | Retinolpalmitat | | | 1,0 |
|---|---|---|---|---|
| B | Konservierungsmittel | q.s. | q.s. | q.s. |
| C | Wasser | Ad 100.0 | Ad 100.0 | Ad 100.0 |

Die Herstellung der Formulierungen erfolgt folgendermaßen:

Phase A und Phase C werden auf 90 °C erwärmt. Anschließend wird Phase A unter Rühren zu Phase C gegeben. Diese Pre-Emulsion wird anschließend homogenisiert, z.B. mit einem Ultra Turrax. Die Emulsion wird unter langsamem Rühren abgekühlt bis auf 40 °C und es wird das Konservierungsmittel zugegeben. Anschließend wird weiter unter Rühren abgekühlt bis auf 30 °C.

**Patentansprüche**

1. Zusammensetzung enthaltend

   A) mindestens ein Ceramid,
   B) mindestens einen Polyglycerincarbonsäureester und
   C) Cholesterol und/oder mindestens ein Cholesterolderivat ausgewählt aus der Gruppe Cholesterolsulfat, Cholesterol Hydrogen Succinate und 7-Dehydrocholesterol,

   **dadurch gekennzeichnet, dass**
   das Gewichtsverhältnis aller enthaltenen Ceramide zur Summe des enthaltenen Cholesterols und/oder des mindestens einen Cholesterolderivates von 1:0,1 bis 1:0,9, bevorzugt 1:0,1 bis 1:0,8, besonders bevorzugt von 1:0,2 bis 1:0,7, beträgt.

2. Zusammensetzung gemäß Anspruch 1, **dadurch gekennzeichnet, dass** das Gewichtsverhältnis aller enthaltenen Ceramide zu allen enthaltenen Polyglycerincarbonsäureestern von 1:1 bis 1:30, bevorzugt von 1:1 bis 1:25, besonders bevorzugt von-1:2 bis 1:20, beträgt.

3. Zusammensetzung gemäß Anspruch 1 oder 2, **dadurch gekennzeichnet, dass** das Cholesterol und/oder mindestens eine Cholesterolderivat in einer Gesamtmenge von 0,1 Gew.-% bis 3,0 Gew.-%, bevorzugt von 0,1 Gew.-% bis 2,0 Gew.-%, besonders bevorzugt von 0,2 Gew.-% bis 1,0 Gew.-%, wobei sich die Gewichtsprozente auf die Gesamtzusammensetzung beziehen, enthalten ist.

4. Zusammensetzung gemäß mindestens einem der vorherigen Ansprüche, **dadurch gekennzeichnet, dass** sie bezogen auf die Gesamtzusammensetzung 20 Gew.-% bis 99 Gew.-%, bevorzugt 40 Gew.-% bis 97 Gew.-%, besonders bevorzugt 50 Gew.-% bis 95 Gew.-%, Wasser enthält.

5. Zusammensetzung gemäß mindestens einem der vorherigen Ansprüche, **dadurch gekennzeichnet, dass** sie bezogen auf die Gesamtzusammensetzung Komponente A), B) und C) in einer Gesamtmenge von 1,0 Gew.-% bis 25 Gew.-%, bevorzugt 2,0, Gew.-% bis 20 Gew.-%, besonders bevorzugt 4,0 Gew.-% bis 15 Gew.-%, enthält.

6. Zusammensetzung gemäß mindestens einem der vorherigen Ansprüche, **dadurch gekennzeichnet, dass** sie mindestens zwei Ceramide, bevorzugt mindestens drei Ceramide, besonders bevorzugt genau drei Ceramide, enthält.

7. Zusammensetzung gemäß mindestens einem der vorherigen Ansprüche, **dadurch gekennzeichnet, dass** das mindestens ein Ceramid ausgewählt ist aus der Gruppe Ceramid NP, Ceramid AP, Ceramid EOP, Ceramid NDS, Ceramid ADS, Ceramid EODS, Ceramid NS, Ceramid AS, Ceramid EOS, Ceramid NH, Ceramid AH und Ceramid EOH, bevorzugt ausgewählt ist aus der Gruppe Ceramide NP, Ceramide AP, Ceramid NS, Ceramide EOP und Ceramide EOS.

8. Zusammensetzung gemäß mindestens einem der vorherigen Ansprüche, **dadurch gekennzeichnet, dass** der Polyglycerincarbonsäureester der Komponente B) aus einem Polyglycerin zusammengesetzt ist, welches einen Polymerisationsgrad von 2,0 bis 25, bevorzugt von 2,5 bis 20, besonders bevorzugt von 3,0 bis 15, aufweist.

9. Zusammensetzung gemäß mindestens einem der vorherigen Ansprüche, **dadurch gekennzeichnet, dass** der Polyglycerincarbonsäureester der Komponente B) aus mindestens einer Carbonsäure ausgewählt aus Fettsäuren, insbesondere aufweisend 12 bis 26, bevorzugt 14 bis 24, besonders bevorzugt 16 bis 22, Kohlenstoffatome, zusammengesetzt ist.

10. Zusammensetzung gemäß mindestens einem der vorherigen Ansprüche, **dadurch gekennzeichnet, dass** der Polyglycerincarbonsäureester der Komponente B) aus Mischungen von mindestens zwei ausgewählt aus Palmitinsäure, Stearinsäure und Behensäure, zusammengesetzt ist, wobei die Summe dieser mindestens 60 Gew.-%, bevorzugt mindesten 75 Gew.-%, noch mehr bevorzugt mindestens 85 Gew.-% bezogen auf alle in der Mischung enthaltenen Fettsäuren ausmacht.

11. Zusammensetzung gemäß mindestens einem der vorherigen Ansprüche, **dadurch gekennzeichnet, dass** er eine Verseifungszahl von 20 bis 199 mg KOH / g, bevorzugt von 30 bis 100 mg KOH / g, besonders bevorzugt von 40 bis 70 mg KOH / g, aufweist.

12. Zusammensetzung gemäß mindestens einem der vorherigen Ansprüche in Form einer kosmetischen, pharmazeutischen oder dermatologischen Formulierung.

13. Zusammensetzung gemäß mindestens einem der Ansprüche 1 bis 4 und 6 bis 12, **dadurch gekennzeichnet, dass** sie bezogen auf die Gesamtzusammensetzung Komponente A), B) und C) in einer Gesamtmenge von 0,01 Gew.-% bis 1,25 Gew.-%, bevorzugt 0,01 Gew.-% bis 1,00 Gew.-%, besonders bevorzugt 0,02 Gew.-% bis 1,00 Gew.-%, enthält.

14. Zusammensetzung gemäß mindestens einem der vorherigen Ansprüche, **dadurch gekennzeichnet, dass** sie einen pH-Wert im Bereich von 4,0 bis 8,0, bevorzugt von 4,5 bis 7,4 besonders bevorzugt von 5,0 bis 7,2, aufweist.

15. Verwendung einer Zusammensetzung gemäß mindestens einem der vorherigen Ansprüche zur Erhöhung der Barrierefunktion der Haut.

Abbildung 1

Interleukin 1α

Abbildung 2

ΔSESM

Abbildung 3

ΔR1-R5: T1-T0

Abbildung 4

ΔR1-R5: T2-T0

Abbildung 5

**Europäisches Patentamt**
**European Patent Office**
**Office européen des brevets**

## EUROPÄISCHER RECHERCHENBERICHT

**Nummer der Anmeldung**

EP 20 20 0973

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 2018/033259 A1 (EVONIK DEGUSSA GMBH [DE]) 22. Februar 2018 (2018-02-22) * Seite 25; Beispiel 10b * ----- | 1,4,6-9, 12,13,15 | INV. A61K8/06 A61K8/37 A61K8/68 A61K8/63 A61Q19/00 A61Q19/08 |
| X | WO 99/29293 A1 (DSM NV [NL]; LAMBERS JOHANNES WILHELMUS JAC [NL]) 17. Juni 1999 (1999-06-17) * Seite 15 * ----- | 1-15 | |
| X | US 2010/184733 A1 (KOREVAAR CORNELIS GERRIT NIJS [NL] ET AL) 22. Juli 2010 (2010-07-22) * Seiten 5,9; Beispiele 9-11 * ----- | 1-15 | |

**RECHERCHIERTE SACHGEBIETE (IPC)**

A61K
A61Q

Der vorliegende Recherchenbericht wurde für alle Patentansprüche erstellt

| Recherchenort | Abschlußdatum der Recherche | Prüfer |
|---|---|---|
| München | 19. März 2021 | Bader, Karl Günther |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 20 20 0973

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

19-03-2021

| Im Recherchenbericht angeführtes Patentdokument | Datum der Veröffentlichung | Mitglied(er) der Patentfamilie | Datum der Veröffentlichung |
|---|---|---|---|
| WO 2018033259 A1 | 22-02-2018 | BR 112019003247 A2 | 16-07-2019 |
| | | CN 109563021 A | 02-04-2019 |
| | | EP 3500550 A1 | 26-06-2019 |
| | | US 2019202771 A1 | 04-07-2019 |
| | | WO 2018033259 A1 | 22-02-2018 |
| WO 9929293 A1 | 17-06-1999 | BR 9807124 A | 25-01-2000 |
| | | CN 1246789 A | 08-03-2000 |
| | | DE 69818242 T2 | 01-07-2004 |
| | | EP 0975325 A1 | 02-02-2000 |
| | | ES 2207023 T3 | 16-05-2004 |
| | | JP 2001510487 A | 31-07-2001 |
| | | KR 20000070718 A | 25-11-2000 |
| | | US 2003059447 A1 | 27-03-2003 |
| | | US 2003215414 A1 | 20-11-2003 |
| | | WO 9929293 A1 | 17-06-1999 |
| US 2010184733 A1 | 22-07-2010 | AT 468100 T | 15-06-2010 |
| | | CA 2665552 A1 | 17-04-2008 |
| | | CN 101511333 A | 19-08-2009 |
| | | EP 2051691 A1 | 29-04-2009 |
| | | ES 2345840 T3 | 04-10-2010 |
| | | JP 5191991 B2 | 08-05-2013 |
| | | JP 2010505886 A | 25-02-2010 |
| | | KR 20090075821 A | 09-07-2009 |
| | | PL 2051691 T3 | 29-10-2010 |
| | | US 2010184733 A1 | 22-07-2010 |
| | | WO 2008043386 A1 | 17-04-2008 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 3117821 A
- EP 2295032 A
- EP 975325 A
- US 8710034 B
- US 2006198800 A1
- DE 102008001788
- US 2006198800 A

**In der Beschreibung aufgeführte Nicht-Patentliteratur**

- **CASSEL et al.** Original synthesis of linear, branched and cyclic oligoglycerol Standards. *J. Org. Chem.,* 2001, 875-896
- **BEISPIEL K. SCHRADER.** Grundlagen und Rezepturen der Kosmetika. Hüthig Buch Verlag Heidelberg, 329-341